Europäisches Patentamt

**European Patent Office**

Office européen des brevets

(19)

(11) Veröffentlichungsnummer: **0 194 600**
A2

# (12) EUROPÄISCHE PATENTANMELDUNG

(21) Anmeldenummer: 86103035.1

(22) Anmeldetag: 07.03.86

(51) Int. Cl.⁴: **C 07 K 15/00**
C 12 P 21/00, C 12 N 15/00
C 12 N 9/99, A 61 K 39/395
//(C12P21/00, C12R1:91)

(30) Priorität: 08.03.85 DE 3508384

(43) Veröffentlichungstag der Anmeldung:
17.09.86 Patentblatt 86/38

(84) Benannte Vertragsstaaten:
AT BE CH DE FR GB IT LI LU NL SE

(71) Anmelder: BOEHRINGER MANNHEIM GMBH
Patentabteilung, Abt. E Sandhofer Strasse 112-132
Postfach 31 01 20
D-6800 Mannheim 31 Waldhof(DE)

(72) Erfinder: Gerber, Martin, Dr. ner. nat.
Ignaz-Manz-Strasse 1
D-8120 Weilheim-Unterhausen(DE)

(72) Erfinder: Naujoks, Kurt Walter, Dr. Dipl.-Biochem.
Hiltlstrasse 15
D-8035 Gauting(DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel Möhlstrasse 22
D-8000 München 80(DE)

(54) Hemmung humaner Speichel- und Pankreasamylase durch monoklonale Antikörper.

(57) Die Erfindung betrifft die Enzymaktivität von humaner
alpha-Amylase zu mehr als 90 % unspezifisch hemmende
monoklonale Anti-alpha-Amylase-Antikörper, ein Verfahren
zu ihrer Herstellung und sie enthaltende Arzneimittel.

EP 0 194 600 A2

Croydon Printing Company Ltd.

/

B e s c h r e i b u n g

Alpha-Amylasen (1,4-alpha-Glucanohydrolasen, EC 3.2.1.1)
spalten 1,4-alpha-glucosidisch verknüpfte Polysaccharide
zu Maltose und Maltooligosacchariden. Beim Menschen
wird alpha-Amylase in den Speicheldrüsen (h-S-A) und im
Pankreas (h-P-A) gebildet. Sie dient dazu, die mit der
Nahrung aufgenommene hochpolymere Stärke zu kleinen
Bruchstücken zu verdauen, die dann durch weitere Enzyme
zu Glucose gespalten wird. Die gebildete Glucose wird
durch die Blutbahn zu den energieverbrauchenden Organen
transportiert.

Der Glucosegehalt des Blutes wird durch Insulin reguliert. Fehlt dieses Hormon - wie bei Patienten, die an
Diabetes mellitus leiden - dann kommt es nach Nahrungsaufnahme zu einer Hyperglykämie. Damit dieser Glucoseschock geschwächt wird und damit die Patienten in der
Nahrungsaufnahme nicht zu sehr eingeschränkt sind, ist
es wünschenswert, die Glucosefreisetzung im Blut zu
verlangsamen. Eine Möglichkeit hierfür wäre die Hemmung
der Speichel- und Pankreasamylase, die die Primärverdauung der Kohlenhydrate bewirken. Die Einstellung des
Zuckerspiegels im Blut der Patienten würde dadurch
wesentlich erleichtert.

Als mögliche Inhibitoren bieten sich Pflanzenproteine
aus z. B. Weizenkeimen (Biochem. Biophys. Acta 658,
397-405 (1981)) und Proteine und Peptide aus Mikroorganismen an (Prepar. Biochem. 9, 293-302 (1979); Agric.
Biol. Chem. 44, 1679-1681 (1980); Hoppe-Seyler's Z.
Physiol. Chem. 362, 465-467 (1981)). Diese zeigen

2

jedoch erhebliche Nachteile, so z. B. eine umständliche und teure Isolierung; außerdem ist eine Vorinkubation von Inhibitor und Amylase nötig, damit die Hemmung zustande kommt.

Aus Comp. Biochem. Physiol. (1976) 55B, S. 563-565 ist bekannt, daß Antiserum aus Kaninchen humane alpha-Amylase bis zu etwa 84% zu hemmen vermag. Diese polyklonalen Antikörper hemmen anscheinend h-P-A und h-S-A. Eine Verwendung derartiger polyklonaler Antikörper als Arzneimittel wurde nicht in betracht gezogen.

Nunmehr wurde überraschenderweise gefunden, daß monoklonale Antikörper gegen alpha-Amylase hergestellt werden können, welche das Enzym unspezifisch zu mehr als 90 % zu hemmen vermögen und sich daher insbesondere für eine Arzneimittelverwendung eignen.

Gegenstand der Erfindung ist daher ein die Enzymaktivität von humaner alpha-Amylase zu mehr als 90 % unspezifisch hemmender monoklonaler Anti-alpha-Amylase-Antikörper.

Unter unspezifisch hemmend wird hier verstanden, daß der Antikörper Speichel-alpha-Amylase und Pankreas-alpha-Amylase in etwa gleichem Ausmaß hemmt, also nicht zwischen diesen beiden Isoenzymen unterscheidet. Diese Eigenschaft ist für die Brauchbarkeit als Arzneimittel wesentlich. Der erfindungsgemäße Antikörper kann auch zur Reinigung sowohl von Speichel- als auch Pankreasamylase verwendet werden, wozu er zweckmäßig in immobilisierter Form eingesetzt wird.

Der erfindungsgemäße monoklonale Antikörper hemmt vorzugsweise die Isoenzyme der humanen alpha-Amylase zu 95% oder mehr. Besonders bevorzugt werden die durch die Klone 77F5 (NCACC 85022203) und 73C8 (NCACC 85022204) gebildeten Antikörper. Die vorstehend angegebenen prozentualen Hemmungswerte beziehen sich auf den handelsüblichen alpha-Amylasetest mit blaugefärbtem hochpolymerem Stärkesubstrat. Bei niedermolekularen Substraten beträgt die entsprechende Hemmwirkung mehr als 80 %. Unter "niedermolekular" werden hier Substrate mit bis zu etwa 10 Glucoseeinheiten verstanden. Die gute Hemmung der Umsetzung niedermolekularer Substrate ist für die therapeutische Verwendung deshalb von Bedeutung, weil die hochmolekularen Substrate von der Speichelamylase ja bereits gespalten werden und deshalb die Pankreasamylase im Darm auch kurzkettige Substrate vorfindet.

Zur Gewinnung des erfindungsgemäßen monoklonalen Antikörpers verwendet man gemäß der Erfindung Balb$_c$-Mäuse, die man mit humaner Speichel-alpha-Amylase immunisiert, dann die B-Lymphocyten aus der Milz der immunisierten Tiere mit Myelomzellen fusioniert, die gebildeten Hybridomazellen kloniert, einen Speichel- und Pankreas-alpha-Amylase vergleichbar hemmenden Klon isoliert und züchtet und den durch den Klon gebildeten monoklonalen Antikörper gewinnt.

Vorzugsweise erfolgt die Immunisierung der Mäuse unter Mitverabreichung von Aluminiumhydroxid und Bordetella Pertussis. Die Immunisierung wird zweckmäßig mehrmals wiederholt. Gute Ergebnisse wurden mit 3- bis 5-facher Wiederholung erhalten. Die Gewinnung der B-Lymphocyten aus der Milz erfolgt nach üblichen Methoden. Die Fusion mit den Myelomzellen wird vorzugsweise nach dem in J. Immol. Meth. 39, 285-308 (1980) beschriebenen Standardverfahren durchgeführt. Die B-Lymphocyten werden hier zweckmäßig im Überschuß eingesetzt.

Die Identifizierung von Klonen, welche einen Antikörper bilden, der die menschlichen Isoenzyme der alpha-Amylase etwa gleich stark hemmt, läßt sich nach dem ELISA-Verfahren unter Verwendung von Anti-Maus-Antikörpern durchführen. Vorzugsweise werden Anti-Maus-Fc-gamma-Antikörper, beispielsweise vom Schaf, verwendet. Durch Inkubation mit den einzelnen Überständen der verschiedenen Klone und anschließende weitere Inkubation mit Speichelamylase-Peroxidase-Konjugat bzw. mit Pankreas-alpha-Amylase-Peroxidase-Konjugat und Nachweis der gebundenen Peroxidaseaktivität mit einem der hierfür bekannten Reagenzien, lassen sich diejenigen Klone feststellen, welche mit beiden Isoenzymen gleich stark reagieren. Diese Klone werden isoliert und gezüchtet. Die Züchtung kann in an sich bekannter Weise erfolgen, entweder in der Zellkultur oder durch intraperitoneale Verabreichung an Mäuse und Gewinnung der monoklonalen Antikörper aus der Aszitesflüssigkeit.

Die reine Isolierung des monoklonalen Antikörpers läßt sich ebenfalls nach üblichen Methoden durchführen, beispielsweise durch Chromatographie über eine Anionenaustauschersäule wie eine mit Diethylaminoethanol-Gruppen modifizierende Cellulose- oder Agarose-Säule.

Der erfindungsgemäße monoklonale Antikörper eignet sich, wie bereits erwähnt, als Arzneimittel zur Hemmung der alpha-Amylase, insbesondere im Rahmen von Diabetes Mellitus. Bei der Verabreichung des erfindungsgemäßen
monoklonalen Antikörpers, die oral erfolgen kann, wird
die Glucosefreisetzung im Blut wesentlich verlangsamt
und daher die Einstellung des Zuckerspiegels im Blut    -
erheblich erleichtert. Ein weiterer Gegenstand der
Erfindung ist daher ein Arzneimittel, welches dadurch
gekennzeichnet ist, daß es als Wirkstoff einen erfindungsgemäßen monoklonalen Antikörper, gegebenenfalls
zusammen mit üblichen therapeutischen Zusatz- oder
Konfektionierungsmitteln enthält. Zweckmäßigerweise
wird beim erfindungsgemäßen Arzneimittel der monoklonale Antikörper in an sich bekannter Weise so eingekapselt, daß er den Magen unverdaut passieren kann und
erst im Dünndarm, wo die Pankreasamylase ihre Kohlehydratspaltungswirkung entfaltet, wirksam wird. Geeignete
Einkapselungsmethoden sind dem Fachmann bekannt und
bedürfen hier keiner näheren Erläuterung.

Die folgenden Beispiele erläutern die Erfindung weiter.


B e i s p i e l    1

Immunisierung von Balb$_c$-Mäusen mit humaner Speichel-
Amylase

Balb$_c$-Mäuse werden mit 100 μg humaner Speichelamylase
in Aluminiumhydroxid mit Bordetella Pertussis immunisiert. In ca. achtwöchigem Rhytmus wird mit je 50 μg
Adjuvanz drei- bis viermal weiterimmunisiert. Vier Tage
vor Fusion wird die letzte Immunisierung mit 50 μg
Speichelamylase in physiologischer Kochsalzlösung
intravenös durchgeführt.

6

Beispiel     2

Fusion der Maus-Milzzellen mit Myelomzellen

Die Fusion der Milzzellen mit Ag8.653 (ATCC CRL 1580)
oder Sp2/O (ATCC CRL 1581)-Myelomzellen wird nach dem
Standardverfahren gemäß J. Immol. Meth. $\underline{39}$, 285-308
(1980) durchgeführt. Das Fusionsverhältnis Milz zu
Myelomzellen ist 5:1. Die Fusionsprodukte werden auf 10
24er- Kulturschalen (von Costar) ausgesät und mit $5.10^4$
Peritonealexsudatzellen pro Kulturnapf gefüttert.
Positive Primärkulturen werden 3 bis 4 Wochen nach
Fusion mit Hilfe eines Fluoreszenz-aktivierten Zellsorters kloniert. Die Zellen werden einzeln in 96er
Costar-Platten abgelegt und mit $2.10^4$ Peritoneal-
exsudat-Zellen gefüttert.


Beispiel     3

Test auf amylasebindende Antikörper

Um positive Primärkulturen zu identifizieren bzw. um
die Konzentration und Spezifität amylasebindender Antikörper im Serum immunisierter Mäuse oder im Kulturüberstand der Hybridzellen oder in Ascites zu erfassen,
wird ein ELISA als Testprinzip verwendet. Dazu werden
96er Platten (Fa. Nunc) mit Schaf-anti-MausFcγ-Antikör-
per beschichtet. Zur Reduktion unspezifischer Bindung
wird mit Rinderserumalbumin (2%ig in physiologischer
Kochsalzlösung) nachbeschichtet. Danach wird mit der
den Antikörper enthaltenden Probe oder mit verschiedenen Verdünnungen derselben inkubiert. Die daran anschließende Inkubation wird entweder mit Speichelamylase-Peroxidase-(POD)-Konjugat oder mit Pankreasamylase-
Peroxidase-(POD)-Konjugat durchgeführt. Die Aktivität

der gebundenen POD wird mit ABTS (2,2'-Azinodi-[3-ethyl-
benzthiazolinsulfonat(6)]) bei pH = 4,4 bestimmt und
die Extinktion direkt als Maß für die gebundenen Mausantikörper genommen. Diejenigen Klone, welche mit
Speichelamylase-POD und Pankreasamylase-POD gleich hohe
Extinktionen ergaben, werden in der Ascitesproduktion
eingesetzt und die monoklonalen Antikörper auf ihre
hemmende Wirkung auf Pankreas- und Speichelamylase hin
untersucht.


B e i s p i e l    4


Produktion des monoklonalen Antikörpers über
Maus-Ascites


Weibliche Balb$_c$-Mäuse werden zweimal mit 0,5 ml Pristan
(EGA-Chemie) intraperitoneal im siebentägigen Abstand
gespritzt. Pro Maus werden je ca. $5.10^6$ Hybridomazellen
der Klone 77F5 oder 73C8 intraperitoneal inokkuliert.
Je nach Zellinie wird nach 8 bis 14 Tagen ein- bis
dreimal Ascites gezapft. Dieser wird über eine DEAE-
Säule gereinigt und man erhält den reinen monoklonalen
Antikörper.


B e i s p i e l    5


Test der Hemmwirkung des monoklonalen Antikörpers


Der Ascites, welcher den zu testenden monoklonalen
Antikörper enthält, wird mit 50 mM Phosphatpuffer,
pH = 7,0 (≙ Lösung A), verdünnt (≙ Lösung B). Die
Hemmwirkung wird an Speichelamylase und Pankreasamylaselösungen der Konzentration ca. 1000 U/l und ca.
2000 U/l (bestimmt mit handelsüblichem Amylasereagenz

8

von Boehringer Mannheim, Kat. Best. Nr. 568589, bei 37°C) getestet. Es werden die Amylaselösungen und jeweils zwei Kontrollen vorgemischt:

I.: 100 µl Amylaselösung + 20 µl Lösung B
II.: 100 µl Amylaselösung + 20 µl Lösung A
III.: 100 µl Lösung A + 20 µl Lösung B

Die Mischungen werden 10 Minuten geschüttelt, danach wird 25 µl der Mischung jeweils zu 1000 µl handelsüblichem Reagenz zur Bestimmung von alpha-Amylase mit 4-Nitrophenylmaltoheptasoid (Boehringer Mannheim, Kat. Best. Nr. 568589) gegeben. Die Aktivität wird nach Anleitung bei 37°C bestimmt. Die Restaktivität berechnet sich nach folgender Formel:

$$\text{Restaktivität(\%)} = \frac{\text{Aktivität I} - \text{Aktivität III}}{\text{Aktivität II}} \times 100$$

Die Restaktivitäten betragen mit humaner Speichel- und humaner Pankreasamylase, sowohl bei einer Aktivität von ca. 1000 U/l, als auch bei einer von ca. 2000 U/l, 15 % bis 20 %, d. h. die Hemmwirkung der MAK's beträgt 80% bis 85%.


B e i s p i e l    6

Test der Hemmwirkung der MAK's mit gefärbter Stärke als Substrat

Die humane Speichelamylase- und humane Pankreasamylaselösungen werden entsprechend Beispiel 5 mit den Ascitesproben, die die zu testenden MAK's enthalten, und den Lösungen A und B gemischt und danach 10 Minuten geschüttelt. 50 µl der Mischungen I, II und III werden in einem handelsüblichen alpha-Amylase-Test mit blaugefärbtem, hochpolymerem Stärkesubstrat (Pharmacia Diagnostics AB, Uppsala, Sweden, Kat. Best. Nr. 93-986-2-1393-02) eingesetzt und entsprechend Anleitung bei 37°C bestimmt. Die Restaktivität berechnet sich entsprechend Beispiel 5. Sie beträgt mit humaner Speichel- und mit humaner Pankreasamylase 5 %. Die Hemmrate mit dem hochpolymeren Substrat beträgt also 95%.

P a t e n t a n s p r ü c h e

1. Die Enzymaktivität von humaner alpha-Amylase zu mehr als 90 % unspezifisch hemmender monoklonaler Anti-alpha-Amylase-Antikörper.

2. Monoklonaler Antikörper nach Anspruch 1, d a d u r c h     g e k e n n z e i c h n e t , daß er zu 95 % oder mehr hemmt.

3. Verfahren zur Gewinnung des monoklonalen Antikörpers nach Anspruch 1 oder 2,    d a d u r c h    g e k e n n z e i c h n e t ,      daß man Mäuse mit alpha-Amylase immunisiert, B-Lymphocyten aus der Milz der immunisierten Tiere mit Myelomzellen fusioniert, die gebildeten Hybridomazellen kloniert, die alpha-Amylase aus Speichel und Pankreas vergleichbar stark hemmenden Klone isoliert und züchtet und den durch sie gebildeten monoklonalen Antikörper gewinnt.

4. Verfahren nach Anspruch 3,     d a d u r c h    g e k e n n z e i c h n e t ,      daß man die Immunisierung unter Verabreichung von Aluminiumhydroxid und Bordetella Pertussis durchführt.

5. Verfahren nach Anspruch 3 oder 4,    d a d u r c h    g e k e n n z e i c h n e t , daß man den isolierten Klon in Zellkultur oder Aszites züchtet.

0194600

6. Arzneimittel, d a d u r c h
g e k e n n z e i c h n e t , daß es als
Wirkstoff einen monoklonalen Antikörper nach
Anspruch 1 oder 2, gegebenenfalls zusammen mit
üblichen therapeutischen Zusatz- und Konfektionierungsmitteln, enthält.

7. Monoklonaler Antikörper 77F5, NCACC 85022203.

8. Monoklonaler Antikörper 73C8, NCACC 85022204.